(19) Europäisches Patentamt
European Patent Office
Office européen des brevets

(11) Veröffentlichungsnummer: 0 002 781
B1

(12) # EUROPÄISCHE PATENTSCHRIFT

(21) Anmeldenummer: 78101758.7

(22) Anmeldetag: 19.12.78

(51) Int. Cl.³: C 07 C 45/29, C 07 C 45/37, B 01 J 35/02, B 01 J 23/72, B 01 J 27/14,// C 07 C 47/198, C 07 C 49/175

(54) Verfahren zur Herstellung von aliphatischen, mit aliphatischen Gruppen verätherten hydroxysubstituierten Carbonylverbindungen

(30) Priorität: 24.12.77 DE 2758124
12.04.78 DE 2815752

(43) Veröffentlichungstag der Anmeldung:
11.07.79 Patentblatt 79/14

(45) Bekanntmachung des Hinweises auf die Patenterteilung:
26.11.80 Patentblatt 80/24

(84) Benannte Vertragsstaaten:
BE CH DE FR GB

(56) Entgegenhaltungen:
DE - A - 1 923 048
US - A - 2 170 855

(73) Patentinhaber: BASF Aktiengesellschaft
Carl-Bosch-Strasse 38
D - 6700 Ludwigshafen (DE)

(72) Erfinder: Dudeck, Christian, Dr.
Odenwaldring 20
D - 6703 Limburgerhof (DE)
Lehmann, Gunter
Bayernstrasse 58
D - 6700 Ludwigshafen (DE)
Petri, Norbert, Dr.
Max-Beckmann-Strasse 17
D - 6710 Frankenthal (DE)
Diem, Hans, Dr.
Feldbergstrasse 63
D - 6800 Mannheim 25 (DE)
Fliege, Werner, Dr.
Authariestrasse 6
D - 6701 Otterstadt (DE)
Meissner, Bernd, Dr.
Dammweg 15
D - 6900 Heidelberg 1 (DE)
Ross, Karl-Heinz, Dr.
Sudetenstrasse 8
D - 6704 Mutterstadt (DE)
Muehlthaler, Wolfgang
Regerstrasse 13
D - 6944 Hemsbach (DE)

EP 0 002 781 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von aliphatischen, mit aliphatischen Gruppen verätherten hydroxysubstituierten Carbonylverbindungen

Die Erfindung betrifft ein neues Verfahren zur Herstellung von aliphatischen, mit aliphatischen Gruppen verätherten Hydroxycarbonylverbindungen durch Oxidation von Hydroxyalkoholen in Gegenwart eines Metallkatalysators, der aus einer oder mehr Schichten von jeweils bestimmtem Gewicht und mit jeweils Teilchen bestimmter Korngröße sowie einer bestimmten Gesamtschichtdicke besteht, wobei als Katalysatormetalle Silber und Kupfer und gegebenenfalls zusätzlich Kupfer/Zinn/Phosphor verwendet werden.

Es ist aus Houben-Weyl, Methoden der Organischen Chemie, Band 7/1, Seiten 166 und 167 bekannt, daß Verbindungen, die außer der primären Alkoholgruppe noch Äthergruppen enthalten, sich meist nur mit sehr mäßigen Ausbeuten zu den Aldehyden dehydrieren lassen. Um Spaltreaktionen zu vermeiden, muß man entweder an der untersten Temperaturgrenze arbeiten oder wie im Falle der Dehydrierung des Tetrahydrofurylcarbinols zum Tetrahydrofuran-2-aldehyd bei einer relativ hohen Temperatur und kürzester Verweilzeit am Katalysator umsetzen. So wird für die Herstellung des Methoxyacetaldehyds ein mit Wasserstoff behandelter Kupferoxidkatalysator und eine Reaktionstemperatur von 300°C verwendet. Auf entsprechende Weise kann man auch den Äthoxy- und Butoxyacetaldehyd herstellen. Allerdings sind die Ausbeuten hier noch schlechter.

Eien Arbeit in Zh. Prikl. Khim. (Leningrad) 1970, Band 43, Seiten 1 132—1 136 (engl. Text, Seite 1 137—1 140) beschreibt die Umsetzung von Methylglykol mit Luft an Silberdrahtspiralen als Katalysator bei Temperaturen von 380 bis 578°C und mit Ausbeuten von 24,3 bis 58 Prozent, bezogen auf den Ausgangsstoff. Entsprechende Umsetzungen mit Butylglykol bei 463 bis 488°C wurden durchgeführt. Reaktionstemperaturen von 465 bis 475°C werden in beiden Fällen als vorteilhaft angesehen. Nachteilig ist bei diesen Verfahren, daß trotz Verwendung von Vakuum und unter Zusatz von gleichen Mengen Stickstoff wie Luft nur maximal 58 Prozent Ausbeute erzielt werden.

Ebenfalls ist bekannt (Houben-Weyl, loc. cit., Band 7/2a, Seiten 699 bis 776), sekundäre Alkohole katalytisch zu Ketonen zu dehydrieren bzw. mit Luft zu oxidieren. als Katalysatoren werden im allgemeinen Hydrierungskatalysatoren auch als Dehydrierungskatalysatoren vorgeschlagen, insbesondere die vorgenannten Katalysatoren der Aldehydsynthesen (loc. cit., Seite 700). Man arbeitet im allgemeinen bei der Dehydrierung in der Gasphase zwischen 180 und 400°C, vorwiegend zwischen 200 und 250°C. Für die Synthese von 1-Methoxy-2-oxo-propan führt die Oxidation mit Chromsäure/Schwefelsäure/Wasser-Gemischen nur zu einer Ausbeute von 29 Prozent (Houben-Weyl, loc. cit., Band 7/2a, Seiten 722 bis 724).

Es ist aus der US-Patentschrift 2 170 855 bekannt, daß man Alkoxyisopropanole in der Gasphase mit Luft in Gegenwart von Metalloxiden wie Silber-, Kupfer-, Nickel- und Kobalt-oxid oder von Metallkatalysatoren wie Kupfer, Kupfer/Chrom, Chrom, Silber, Kobalt und Nickel zu Alkoxyacetonen oxidiert. Als Reaktionstemperaturen werden 250 bis 300°C angegeben. Führt man das Verfahren in großtechnischem Maßstab durch, erhält man Ausbeuten von 9,8 bis 33,7 Prozent.

Alle diese Verfahren sind im Hinblick auf einfache und wirtschaftliche Arbeitsweise, Ausbeute und Reinheit des Endstoffs unbefriedigend.

Es wurde nun gefunden, daß man aliphatische, mit aliphatischen Gruppen verätherte Hydroxycarbonylverbindungen durch katalytische Oxidation von Hydroxyalkoholen in Gegenwart von Metallkatalysatoren vorteilhaft erhält, wenn man aliphatische, mit aliphatischen Gruppen verätherte Hydroxyalkohole in Gegenwart eines Katalysators mit der Gesamtschichtdicke von 5 bis 100 Millimeter und

a) 3 oder mehr Schichten Silberkristallen, wobei ein Teil der Schichten Silberkristalle 30 bis 85 Gewichtsprozent des Katalysators mit Teilchen der Korngröße 1 bis 2,5 mm, ein Teil der Schichten silberkristalle 2 bis 30 Gewichtsprozent des Katalysators mit Teilchen der Korngröße 0,75 bis 1 mm und der restliche Teile der Schichten Silberkristalle 8 bis 50 Gewichtsprozent des Katalysators mit Teilchen der Korngröße 0,2 bis 0,75 mm enthalten, und einer oder mehr Schichten Kupferkristallen, die 1 bis 40 Gewichtsprozent des Katalysators mit Teilchen der Korngröße 0,2 bis 0,75 mm enthalten, oder

b) 3 oder mehr Schichten Silberkristallen, wobei ein Teil der Schichten Silberkristalle 30 bis 85 Gewichtsprozent des Katalysators mit Teilchen der Korngröße 1 bis 2,5 mm, ein Teil der Schichten Silberkristalle 2 bis 30 Gewichtsprozent des Katalysators mit Teilchen der Korngröße 0,75 bis 1 mm und der restliche Teil der Schichten Silberkristalle 8 bis 50 Gewichtsprozent des Katalysators mit Teilchen der Korngröße 0,2 bis 0,75 mm enthalten, und einer oder mehr Schichten Kupferkristallen, die 0,5 bis 40 Gewichtsprozent des Katalysators mit Teilchen der Korngröße 0,2 bis 0,75 mm enthalten, und einer oder mehr Schichten von Katalysatorteilchen mit 70 bis 99 Gewichtsprozent Kupfer, 0,9 bis 20 Gewichtsprozent Zinn und 0,1 bis 10 Gewichtsprozent Phosphor, bezogen auf das Gewicht dieser Teilchen an diesen Elementen, wobei diese Schichten Katalysatorteilchen 0,5 bis 10 Gewichtsprozent des Katalysators mit Teilchen der Korngröße 0,2 bis 0,75 mm enthalten, oder

c) 3 oder mehr Schichten Silberkristallen, wobei ein Teil der Schichten Silberkristalle 30 bis 85 Gewichtsprozent des Katalysators mit Teilchen der Korngröße 1 bis 2,5 mm, ein Teil der Schichten Silberkristalle 2 bis 30 Gewichtsprozent des Katalysators mit Teilchen der

Korngröße 0,75 bis 1 mm und der restliche Teil der Schichten Silberkristalle 13 bis 68 Gewichtsprozent des Katalysators mit Teilchen der Korngröße 0,2 bis 0,75 mm enthalten, bei einer Temperatur von 450 bis 700°C oxidiert.

Die Umsetzung kann für den Fall der Verwendung von Methylglykol durch die folgenden Formeln wiedergegeben werden:

$$2\ CH_3O—CH_2—CH_2OH + O_2 \rightarrow 2\ CH_3O—CH_2—CHO + 2\ H_2O$$

Die Umsetzung kann für den Fall der Verwendung von Propylen-(1,2)-glykolmonomethyläther (1-Methoxy-2-hydroxypropan) durch die folgenden Formeln wiedergegeben werden:

$$2CH_3O—CH_2—CHOH—CH_3 + O_2 \rightarrow 2CH_3O—CH_2—CO—CH_3 + 2H_2O$$

Im Vergleich zum Stand der Technik liefert das Verfahren nach der Erfindung überraschend auf einfacherem und wirtschaftlicherem Wege ein besseres Gesamtergebnis mit Bezug auf Ausbeute, Raum-Zeit-Ausbeute und Reinheit des Endstoffs sowie Lebensdauer des Katalysators. Die Lebensdauer des Katalysators beträgt in der Regel mindestens 30 Tage im Falle von Methoxyacetaldehyd. Alle diese vorteilhaften Ergebnisse sind im Hinblick auf den Stand der Technik, insbesondere auf die Lehre von Houben-Weyl, überraschend, denn man hätte angesichts der hohen erfindungsgemäßen Temperaturen zumindest eine wesentliche Verschlechterung der Ausbeute und erhebliche Bildung von Zersetzungsprodukten erwarten sollen. Im Hinblick auf das in Zh. Prikl. Khim. beschriebene Verfahren brauchen Wasser oder Inertgas nicht zugesetzt zu werden. Die aufwendige Arbeitsweise unter Vakuum und mit spezieller Katalysatorbehandlung wird vermieden. Hohe spezifische Katalysatorbelastungen, z.B. 0,4—2,5 t/m² Katalysatorbettqueschnitt · Stunde gegenüber 0,15 t/m² Katalysatorbettquerschnitt · Stunde (Zh. Prikl. Khim.), können erzielt werden. Im Vergleich zu dem in der US-Patentschrift 2 170 855 beschriebenen Verfahren sind Ausbeute und Umsatz höher.

Als aliphatische, mit aliphatischen Gruppen verätherte Hydroxycarbonylverbindungen kommen zweckmäßig solche Endstoffe der Formel

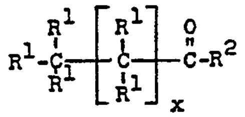

I

und dementsprechend als aliphatische, mit aliphatischen Gruppen verätherte Hydroxyalkohole solche Ausgangsstoffe der Formel

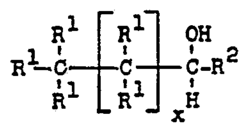

II

in Betracht, worin die einzelnen Reste $R^1$ gleich oder verschieden sein können und jeweils ein Wasserstoffatom, einen aliphatischen Rest oder den Rest $R^3$—O— bedeuten, wobei mindestens ein Rest $R^1$ den Rest $R^3$—O— bezeichnet, $R^2$ für ein Wasserstoffatom oder den Rest

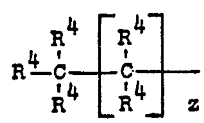

steht, x und z gleich oder verschieden sein können und jeweils 0 oder eine ganze Zahl bedeuten, $R^3$ einen aliphatischen Rest bezeichnet, die einzelnen Reste $R^4$ gleich oder verschieden sein können und jeweils ein Wasserstoffatom, einen aliphatischen Rest oder den Rest $R^3$—O— bedeuten, worin $R^3$ die vorgenannte Bedeutung hat. Bevorzugte Ausgangsstoffe II und dementsprechend bevorzugte Endstoffe I sind solche, in deren Formeln die einzelnen Reste $R^1$ gleich oder verschieden sein können und jeweils ein Wasserstoffatom oder einen Alkylrest mit 1 bis 5 Kohlenstoffatomen oder den Rest $R^3$—O— bedeuten, wobei mindestens ein Rest $R^1$ den Rest $R^3$—O— bezeichnet, $R^2$ für ein Wasserstoffatom oder den Rest

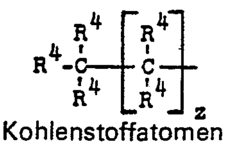

steht, $R^3$ einen Alkylrest mit 1 bis 5 Kohlenstoffatomen bezeichnet, $R^4$ für ein Wasserstoffatom oder einen Alkylrest mit 1 bis 5 Kohlenstoffatomen oder den Rest $R^3$—O—, worin $R^3$ die vorgenannte bevorzugte Bedeutung besitzt, steht, x und z gleich oder verschieden sein können und jeweils 0 oder eine ganze Zahl, insbesondere 0, 1 oder 2, bedeuten. Bedeuten $R^2$ ein Wasserstoffatom, x die Zahl 0,

ein Rest $R^1$ den Rest $R^3$—O— und ein weiterer Rest $R^1$ ein Wasserstoffatom, so bezeichnet der dritte Rest $R^1$ im Falle der Ausführungsform c) bevorzugt einen aliphatischen Rest. Die vorgenannten Reste können noch durch unter den Reaktionsbedingungen inerte Gruppen, z.B. Alkylgruppen mit 1 bis 4 Kohlenstoffatomen, substituiert sein.

Es kommen z.B. als Ausgangsstoffe II in Frage: Methyl-, Äthyl-, n-Propyl-, Isopropyl-, n-Butyl-, sek.-Butyl-, Isobutyl-, tert.-Butyl-äthylenglykol; entsprechende Propylen-(1,2)-, Propylen-(1,3)-, Butylen-(1,2)-, Butylen-(1,3)-, Butylen-(2,3)-, Butylen-(1,4)-, Isobutylen-, Pentylen-(1,5)-, Hexylen-(1,6)-glykoläther; in einer $\alpha$-Stellung zur Hydroxylgruppe durch 2 oder 3 Methoxygruppen substituiertes Äthanol, n-Propanol, 2-Hydroxypropan, n-Butanol, 2-Hydroxybutan, Isobutanol und entsprechende Di- bzw. Triäthyl-($\alpha$)-äther; in $\alpha$-, $\alpha'$-, $\beta$-, $\beta'$-, $\alpha,\alpha'$-, $\beta,\beta'$-, $\alpha,\beta'$-, $\beta,\alpha'$-Stellung zur Hydroxygruppe entsprechend mehrfach oder vorteilhaft einfach, zweifach oder dreifach durch die Methoxy-, Äthoxy-, Propoxy-, Isopropoxy-, Butoxy-, tert.-Butoxy-, sek.-Butoxy-, Isobutoxy-gruppe substituiertes 2-Hydroxypropan, 2-Hydroxybutan, 2-Hydroxypentan, 3-Hydroxypentan.

Als oxidierendes Agens lassen sich sowohl der reine Sauerstoff als auch freien Sauerstoff enthaltende Gase, insbesondere Luft, verwenden. Sauerstoff, in der Regel in Gestalt von Luft, und Ausgangsstoff II werden zweckmäßig imn Molverhältnis von 0,25 bis 0,8, insbesondere von 0,35 bis 0,6 Mol Sauerstoff je Mol Ausgangsstoff II angewandt. Eine Verwendung von Inertgas ist nicht notwendig, stört jedoch andererseits auch nicht die Umsetzung. Gegebenenfalls kann man mit heißen Inertgasen, zweckmäßig Stickstoff oder rußarmen Verbrennungsgasen, die keine Katalysatorgifte enthalten, z.B. von einer Temperatur von 600 bis 800°C, den Katalysator erhitzen.

Die Gesamtschichtdicke des Katalysators beträgt 5 bis 100, vorzugsweise 10 bis 30 mm. Die Katalysatorteilchen in Gestalt von Silberkristallen befinden sich im Katalysator des üblicherweise vertikal aufgestellten Reaktors je nach Korngröße in einem oberen, mittleren oder unteren Teil der Gesamtschicht angeordnet. Das Ausgangsgemisch aus Dampf des Ausgangsstoffes II und Sauerstoff bzw. Luft wird im allgemeinen von oben nach unten geführt, so daß die obere Schicht (obere Schichten) gleichzeitig den dem Ausgangsgemisch zugewandten Teil bedeutet. Bei Reaktoren anderer Bauart oder anderer Führung des Ausgangsgemisches gelten sinngemäß alle Angaben der Beschreibung über oberen (unteren) Teil das Katalysators für den entsprechenden, dem ausgangsgemisch (dem abgeführten Reaktionsgemisch) zugewandten Teil, z.B. bei horizontal angeordneten Reaktoren für den vorderen (hinteren) Teil des Katalysators. Die folgenden Angaben bezüglich dem Anteil in Gewichtsprozent aller Katalysatorteilchen beziehen sich auf das Gesamtgewicht des Katalysators, d. h. sowohl im Falle der Ausführungsform a) der Silberkristalle und Kupferkristalle wie auch im Falle der Ausführungsform b) der Silberkristalle und Kupferkristalle und der Kupfer/Zinn/Phosphor-Katalysatorteilchen (Cu/Sn/P-Teilchen) wie auch im Falle der Ausführungsform c) der Silberkristalle allein.

Folgende Katalysatoren verwendet man bei der Ausführungsform a): Im unteren Teil der Silberkristalle befinden sich 30 bis 85, vorzugsweise 50 bis 78 Gewichtsprozent aller Katalysatorteilchen, im mittleren Teil der Silberkristalle 2 bis 30, vorzugsweise 5 bis 25 Gewichtsprozent aller Katalysatorteilchen, im oberen Teil der Silberkristalle 8 bis 50, vorzugsweise 13 bis 35 Gewichtsprozent aller Katalysatorteilchen. Die Teilchen des unteren Schichtteils haben Korngrößen von 1 bis 2,5, die des mittleren Schichtteils von 0,75 bis 1, die des oberen Schichtteils 0,2 bis 0,75 mm. Jeder Silberschichtteil kann aus einer oder mehreren Schichten, vorzugsweise aus 1, 2 oder 3 Schichten bestehen. Bevorzugt ist ein 4- bis 7-Silberschichtenkatalysator, insbesondere ein 4- oder 5-Silberschichtenkatalysator. Jede dieser Schichten unterscheidet sich von der anderen in der Korngröße der Silberkristalle und meistens auch im zugehörigen Gewichtsanteil des Gesamtkatalysators.

Hat der obere Silberschichtteil 2 Silberschichten, so haben seine untere Silberschicht bevorzugt einen Anteil von 4 bis 46 Gewichtsprozent und Teilchen einer Korngröße von 0,4 bis 0,75 mm und seine obere Silberschicht entsprechend einen Gewichtsanteil von 4 bis 46 Gewichtsprozent und Teilchen der Korngröße von 0,2 bis 0,4 mm. Sind 3 Silberschichten im oberen Silberschichtteil vorhanden, so sind mit Bezug auf Gewichtsanteil am Gesamtkatalysator (Korngröße der Teilchen) bevorzugt: obere Silberschicht 3 bis 45 (0,2 bis 0,4 mm); mittlere Silberschicht 3 bis 45 (0,4 bis 0,6 mm); untere Silberschicht 2 bis 44 (0,6 bis 0,75 mm) Gewichtsprozent. Entsprechend sind bei dem mittleren Silberschichtteil mit Bezug auf Gewichtsanteil (Korngröße der Teilchen) bevorzugt:

a) 2 Silberschichten: obere Silberschicht 1 bis 29 (0,75 bis 0,9 mm) Gew.%;
untere Silberschicht 1 bis 29 (0,9 bis 1 mm) Gew.%.

b) 3 Silberschichten: obere Silberschicht 0,7 bis 28,7 (0,75 bis 0,8 mm) Gew.%;
mittlere Silberschicht 0,7 bis 28,7 (0,8 bis 0,9 mm) Gew.%;
untere Silberschicht 0,6 bis 28,6 (0,9 bis 1 mm) Gew.%.

Bei dem unteren Silberschichtteil sind bevorzugt:

c) 2 Silberschichten: obere Silberschicht 15 bis 70 (1 bis 1,75 mm) Gew.%;
untere Silberschicht 15 bis 70 (1,75 bis 2,5 mm) Gew.%.

d) 3 Silberschichten: obere Silberschicht 10 bis 65 (1 bis 1,5 mm) Gew.%;
mittlere Silberschicht 10 bis 65 (1,5 bis 2 mm) Gew.%;
untere Silberschicht 10 bis 65 (2 bis 2,5 mm) Gew.%.

Die Kupferkristalle befinden sich im Katalysator ebenfalls in Schichten angeordnet. Die Zahl der Kupferkristallschichten kann, bezogen auf die Zahl der Silberkristallschichten, größer, gleich oder bevorzugt kleiner sein. Vorteilhaft sind eine bis 2 Kupferschichten, vorzugsweise eine Kupferschicht. Wird nur eine Kupferschicht verwendet, so enthält sie im Falle a) 1 bis 40, im Falle b) 0,5 bis 40, vorzugsweise in beiden Fällen 2 bis 30 Gewichtsprozent aller Katalysatorteilchen mit Korngrößen von 0,2 bis 0,75 mm. Verwendet man 2 Kupferschichten, dann befinden sich zweckmäßig im unteren Teil 0,5 bis 39,5, vorzugsweise 1 bis 29 Gewichtsprozent aller Katalysatorteilchen und im oberen Teil 0,5 bis 39,5, vorzugsweise 1 bis 29 Gewichtsprozent aller Katalysatorteilchen. Die Teilchen des unteren Schichtteils haben Korngrößen von 0,4 bis 0,75, die des oberen Schichtteils 0,2 bis 0,4 mm.

Die Kupferkristallschichten können einzeln oder mehrere zusammen oberhalb und/oder unterhalb jeder Silberkristallschicht angeordnet sein. Zweckmäßig verwendet man nur eine oder 2 Kupferschichten und legt sie oberhalb und/oder unterhalb des oberen Silberschichtteils, Hat der obere Silberschichtteil mehrere, zweckmäßig 2 oder 3 Silberschichten, so verwendet man bevorzugt 2 Kupferschichten, die man oberhalb und unterhalb der obersten Silberschicht des oberen Silberschichtteils legt, oder vorteilhafter nur eine Kupferschicht (von oben nach unten gezählt), die über der obersten Silberschicht liegt.

Die Schichtung jeder einzelnen Silber- oder Kupferschicht und gegebenenfalls auch jeder Cu/Sn/P-Teilchenschicht ist meist regelmäßig, so daß die Schichtdicke der Einzelschicht über den ganzen Schichtquerschnitt hinweg gleich ist. In diesen Fällen hängt die Schichtdicke direkt von den vorgenannten Gewichtsanteilen Gesamtkatalysator und der jeweiligen Korngröße der Teilchen ab. Man kann aber auch eine unregelmäßige Schichtung aller oder mehrerer Schichten oder zweckmäßig einer Silber- oder Kupferschicht oder Cu/Sn/P-Teilchenschicht vornehmen, z.B. in der Mitte, auf den Seiten oder vorteilhaft am Rande der Schicht die Hauptmenge der katalysatorteilchen aufgeben und entsprechend nur eine kleinere Restmenge auf die übrige Schicht verteilen. In einer bevorzugten Ausführungsform werden mehrere Einzelschichten oder vorteilhaft eine Einzelschicht nur am Rande der Katalysatorzone in Gestalt einer ring- oder kranzförmigen Schicht mit ebener Ober- und Unterseite der Schicht (Ringschicht) auf die jeweils darunterliegende, regelmäßig angeordnete Schicht mit regelmäßiger Schichtdicke aufgegeben. Vorteilhaft ist folgende Anordnung: Der oberen Schicht (Schicht 1) des Katalysators wird am Rande eine Ringschicht aufgesetzt; zweckmäßig beträgt der Durchmesser der ringförmigen Schicht, d. h. die Differenz von Durchmesser des Querschnitts des Gesamtkatalysators und der lichten Weite des Schichtringes, den 100sten bis den 10ten Teil des Katalysatordurchmessers und damit des Durchmessers der oberen, regelmäßigen, Schicht. Besonders bevorzugt ist die Anordnung, eine solche Ringschicht der oberen Schicht (Schicht 1) nicht aufzusetzen sondern zu unterlegen und somit der darunterliegenden Schicht (Schicht 2) aufzusetzen. Auf diese Weise gewinnen Ringschicht und Schicht 1 bzw. Ringschicht, Schicht 1 und Schicht 2 die Form einer flachen Schale mit nach oben gewölbtem Rand. Enthält der obere Schichtteil mehrere, z.B. 2 oder 3 Schichten, so kann die Ringschicht jeder Schicht des oberen Schichtteils entsprechend unterlegt werden, z.B. unter Schicht 2 oder 3. Da als Reaktoren üblicherweise Reaktionsrohr bzw. rohrförmige Reaktionsräume verwendet werden, liegt ein solcher Rand am äußeren Rohrkranz des Katalysatorträgers bzw. an der inneren Rohrwand.

Ein besonders vorteilhafter Katalysator im Falle der Ausführungsform a) hat die folgende Zusammensetzung, wobei Schicht 3 als Ringschicht Silberkristalle mit einem Ringdurchmesser, der dem 60sten Teil des Katalysatordurchmessers entspricht, ausgebildet und Schicht 2 unterlegt und somit Schicht 4 aufgesetzt ist:

Schicht 1 (Kupferschicht): 10,7 Gew.% des Katalysators mit Teilchen der Korngröße 0,2—0,4 mm

Schicht 2 (Kupferschicht): 10,3 Gew.% des Katalysators mit Teilchen der Korngröße 0,4—0,75 mm

Schicht 3 (Silberschicht): 13,4 Gew.% des Katalysators mit Teilchen der Korngröße 0,2—0,75 mm

Schicht 4 (Silberschicht): 5,3 Gew.% des Katalysators mit Teilchen der Korngröße 0,75—1 mm

Schicht 5 (Silberschicht): 60,3 Gew.% des Katalysators mit Teilchen der Korngröße 1—2,5 mm. (unterste)

Die bevorzugte Ausführungsform ist b). Hier werden 3 oder mehr, vorzugsweise 3 bis 9, Silberschichten, eine oder mehr Kupferschichten, vorzugsweise eine Kupferschicht und eine oder mehr

Cu/Sn/P-Teilchenschichten, vorzugsweise eine Cu/Sn/P-Teilchenschicht zur Herstellung des Gesamtkatalysators verwendet.

In der Ausführungsform b) werden eine oder mehr Schichten (Cu/Sn/P-Teilchenschichten) von Katalysatorteilchen mit 70 bis 99 Gewichtsprozent Kupfer, 0,9 bis 20 Gewichtsprozent Zinn und 0,1 bis 10 Gewichtsprozent Phosphor, bezogen auf das Gewicht dieser Teilchen an diesen Elementen, verwendet, vorteilhaft 1 bis 2 Schichten, insbesondere eine Schicht. Zweckmäßig legt man ein oder 2 Cu/Sn/P-Teilchenschichten dem Gesamtkatalysator als oberste Schicht bzw. oberste 2 Schichten auf. Bevorzugt sind Katalysatorteilchen, die 90 bis 97 Gewichtsprozent Kupfer, 2,9 bis 7 Gewichsprozent Zinn und 0,1 bis 3 Gewichtsprozent Phosphor, bezogen auf das Gewicht dieser Teilchen an diesen Elementen, enthalten. Die Metalle können als solche oder als Oxide, der Phosphor als Phosphorsäure, Phosphat oder als Oxid vorliegen. Entsprechend können die Teilchen als Gemenge vorgenannter Einzelstoffe und/oder als Gemisch von Verbindungen miteinander wie Phosphorsäure, Kupferoxid, Zinnoxid, vorliegen; vorteilhaft werden sie in üblicher Weise, z.B. durch Vermischen der Komponenten, vorteilhaft vorgenannter Verbindungen, in einem Kneter, hergestellt. Die vorgenannten Gewichtsprozente der 3 Komponenten errechnen isch somit aus dem Gehalt der Teilchen an diesen 3 Elementen und beziehen sich auf das Gesamtgewicht der Teichen an diesen 3 Elementen, ohne Bezug auf die tatsächliche Konstitution der Verbindungen, die das Teichen ergeben. Verwendet man nur eine Schicht, so hat diese 0,5 bis 10, bevorzugt 2 bis 6 Gewichtsprozent des Katalysators mit Teilchen der Korngröße 0,2 bis 0,75 mm. Im Falle von 2 Schichten Katalysatorteilchen befinden sich im unteren Teil 0,25 bis 9,75, vorzugsweise 1 bis 5 Gewichtsprozent aller Katalysatorteilchen und im oberen Teil 0,25 s 9,75, vorzugsweise 1 bis 5 Gewichtsprozent aller Katalysatorteilchen. Die Teilchen des unteren Schichtteils haben Korngrößen von 0,4 bis 0,75, die des oberen Schichtteils 0,2 bis 0,4 mm. Bezüglich Schichtteile, Schichtenzahl, Schichtung, Gewichtsanteile und Korngrößen der Silber- und Kupferschichten gelten die vorgenannten allgemeinen und bevorzugten Werte und Angaben. Bevorzugt sind die folgenden Schichtanordnungen:

| Schichtenzahl | Schichten (von oben nach unten) gezählt | enthält als Kristalle bzw. Teilchen Ag oder Cu oder Cu/Sn/P | Gew. % des Gesamtkatalysators | | Korngröße in mm | |
|---|---|---|---|---|---|---|
| | | | von | bis | von | bis |
| 5 | 1 | Cu/Sn/P | 0,5 | 10 | 0,2 | 0,75 |
| | 2 | Cu | 0,5 | 40 | 0,2 | 0,75 |
| | 3 | Ag | 8 | 50 | 0,2 | 0,75 |
| | 4 | Ag | 2 | 30 | 0,75 | 1 |
| | 5 | Ag | 30 | 85 | 1 | 2,5 |
| 6 | 1 | Cu/Sn/P | 0,5 | 10 | 0,2 | 0,75 |
| | 2 | Cu | 0,5 | 40 | 0,2 | 0,75 |
| | 3 | Ag | 8 | 50 | 0,2 | 0,75 |
| | 4 | Ag | 2 | 30 | 0,75 | 1 |
| | 5 | Ag | 15 | 70 | 1 | 1,75 |
| | 6 | Ag | 15 | 70 | 1,75 | 2,5 |
| 7 | 1 | Cu/Sn/P | 0,5 | 10 | 0,2 | 0,75 |
| | 2 | Cu | 0,5 | 40 | 0,2 | 0,75 |
| | 3 | Ag | 8 | 50 | 0,2 | 0,75 |
| | 4 | Ag | 1 | 29 | 0,75 | 0,9 |
| | 5 | Ag | 1 | 29 | 0,9 | 1,0 |
| | 6 | Ag | 15 | 70 | 1 | 1,75 |
| | 7 | Ag | 15 | 70 | 1,75 | 2,5 |

| Schichtenzahl | Schichten (von oben nach unten) gezählt | enthält als Kristalle bzw. Teilchen Ag oder Cu oder Cu/Sn/P | Gew. % des Gesamtkatalysators von | bis | Korngröße in mm von | bis |
|---|---|---|---|---|---|---|
| 8 | 1 | Cu/Sn/P | 0,5 | 10 | 0,2 | 0,75 |
| | 2 | Cu | 0,5 | 40 | 0,2 | 0,75 |
| | 3 | Ag | 8 | 50 | 0,2 | 0,75 |
| | 4 | Ag | 1 | 29 | 0,75 | 0,9 |
| | 5 | Ag | 1 | 29 | 0,9 | 1 |
| | 6 | Ag | 10 | 65 | 1 | 1,5 |
| | 7 | Ag | 10 | 65 | 1,5 | 2 |
| | 8 | Ag | 10 | 65 | 2 | 2,5 |
| 9 | 1 | Cu/Sn/P | 0,5 | 10 | 0,2 | 0,75 |
| | 2 | Cu | 0,5 | 40 | 0,2 | 0,75 |
| | 3 | Ag | 8 | 50 | 0,2 | 0,75 |
| | 4 | Ag | 0,7 | 28,7 | 0,75 | 0,8 |
| | 5 | Ag | 0,7 | 28,7 | 0,8 | 0,9 |
| | 6 | Ag | 0,6 | 28,6 | 0,9 | 1 |
| | 7 | Ag | 10 | 65 | 1 | 1,5 |
| | 8 | Ag | 10 | 65 | 1,5 | 2 |
| | 9 | Ag | 10 | 65 | 2 | 2.5 |

| Schichtenzahl | Schichten (von oben nach unten) gezählt | enthält als Kristalle bzw. Teilchen Ag oder Cu oder Cu/Sn/P | Gew. % des Gesamtkatalysators | | Korngrößr in mm | |
|---|---|---|---|---|---|---|
| | | | von | bis | von | bis |
| | 1 | Cu/Sn/P | 0.5 | 10 | 0,2 | 0,75 |
| | 2 | Cu | 0,5 | 40 | 0,2 | 0,75 |
| | 3 | Ag | 4 | 46 | 0,2 | 0,4 |
| | 4 | Ag | 4 | 46 | 0,4 | 0,75 |
| 10 | 5 | Ag | 0,7 | 28,7 | 0,75 | 0,8 |
| | 6 | Ag | 0,7 | 28,7 | 0,8 | 0,9 |
| | 7 | Ag | 0,6 | 28,6 | 0,9 | 1 |
| | 8 | Ag | 10 | 65 | 1 | 1,5 |
| | 9 | Ag | 10 | 65 | 1,5 | 2 |
| | 10 | Ag | 10 | 65 | 2 | 2,5 |
| | 1 | Cu/Sn/P | 0,5 | 10 | 0,2 | 0,75 |
| | 2 | Cu | 0,5 | 40 | 0,2 | 0,75 |
| | 3 | Ag | 3 | 45 | 0,2 | 0,4 |
| | 4 | Ag | 3 | 45 | 0,4 | 0,6 |
| 11 | 5 | Ag | 2 | 44 | 0,6 | 0,75 |
| | 6 | Ag | 0,7 | 28,7 | 0,75 | 0,8 |
| | 7 | Ag | 0,7 | 28,7 | 0,8 | 0,9 |
| | 8 | Ag | 0,6 | 28,6 | 0,9 | 1 |
| | 9 | Ag | 10 | 65 | 1 | 1,5 |
| | 10 | Ag | 10 | 65 | 1,5 | 2 |
| | 11 | Ag | 10 | 65 | 2 | 2,5 |

| Schichtenzahl | Schichten (von oben nach unten) gezählt | enthält als Kristalle bzw. Teilchen Ag oder Cu oder Cu /Sn /P | Gew. % des Gesamtkatalysators | | Korngröße in mm | |
|---|---|---|---|---|---|---|
| | | | von | bis | von | bis |
| 12 | 1 | Cu /Sn /P | 0,5 | 10 | 0,2 | 0,75 |
| | 2 | Cu | 0,5 | 39,5 | 0,2 | 0,4 |
| | 3 | Cu | 0,5 | 39,5 | 0,4 | 0,75 |
| | 4 | Ag | 3 | 45 | 0,2 | 0,4 |
| | 5 | Ag | 3 | 45 | 0,4 | 0,6 |
| | 6 | Ag | 2 | 44 | 0,6 | 0,75 |
| | 7 | Ag | 0,7 | 28,7 | 0,75 | 0,8 |
| | 8 | Ag | 0,7 | 28,7 | 0,8 | 0,9 |
| | 9 | Ag | 0,6 | 28,6 | 0,9 | 1 |
| | 10 | Ag | 10 | 65 | 1 | 1,5 |
| | 11 | Ag | 10 | 65 | 1,5 | 2 |
| | 12 | Ag | 10 | 65 | 2 | 2,5 |

| Schichtenzahl | Schichten (von oben nach unten) gezählt | enthält als Kristalle bzw. Teilchen Ag oder Cu oder Cu /Sn /P | Gew. % des Gesamtkatalysators | | Korngröße in mm | |
|---|---|---|---|---|---|---|
| | | | von | bis | von | bis |
| 13 | 1 | Cu /Sn /P | 0,25 | 9,75 | 0,2 | 0,4 |
| | 2 | Cu /Sn /P | 0,25 | 9,75 | 0,4 | 0,75 |
| | 3 | Cu | 0,5 | 39,5 | 0,2 | 0,4 |
| | 4 | Cu | 0,5 | 39,5 | 0,4 | 0,75 |
| | 5 | Ag | 3 | 45 | 0,2 | 0,4 |
| | 6 | Ag | 3 | 45 | 0,4 | 0,6 |
| | 7 | Ag | 2 | 44 | 0,6 | 0,75 |
| | 8 | Ag | 0,7 | 28,7 | 0,75 | 0,8 |
| | 9 | Ag | 0,7 | 28,7 | 0,8 | 0,9 |
| | 10 | Ag | 0,6 | 28,6 | 0,9 | 1 |
| | 11 | Ag | 10 | 65 | 1 | 1,5 |
| | 12 | Ag | 10 | 65 | 1,5 | 2 |
| | 13 | Ag | 10 | 65 | 2 | 2,5 |

Insbesondere bevorzugt sind im Falle der Ausführungsform a) und b) vorgenannte Katalysatoren mit 5, 6 und 7 Schichten.

Folgende Katalysatoren verwendet man bei der Ausführungsform c): Im unteren Teil der Silberkristalle befinden sich 30 bis 85, vorzugsweise 50 bis 78 Gewichtsprozent aller Katalysatorteilchen, im mittleren Teil der Silberkristalle 2 bis 30, vorzugsweise 5 bis 25 Gewichtsprozent aller Katalysatorteilchen, im oberen Teil der Silberkristalle 13 bis 68, vorzugsweise 17 bis 45 Gewichtsprozent aller Katalysatorteilchen. Die Teilchen des unteren Schichtteils haben Korngrößen von 1 bis 2,5, die des mittleren Schichtteils von 0,75 bis 1, die des oberen Schichtteils 0,2 bis 0,75 mm. Jeder Silberschichtteil kann aus einer oder mehreren Schichten, vorzugsweise aus 1, 2 oder 3 Schichten bestehen. Bevorzugt ist ein 4- bis 7-Silberschichterkatalysator, insbesondere ein 4- oder 5-Silberschichtenkatalysator. Jede dieser Schichten unterscheidet sich von der anderen in der Korngröße der Silberkristalle und meistens auch im zugehörigen Gewichtsanteil des Gesamtkatalysators.

Hat der obere Silberschichtteil 2 Silberschichten, so haben seine untere Silberschicht bevorzugt einen Anteil von 6,5 bis 61,5 Gewichtsprozent und Teilchen einer Korngröße von 0,4 bis 0,75 mm und seine obere Silberschicht entsprechend einen Gewichtsanteil von 6,5 bis 61,5 Gewichtsprozent und Teilchen der Korngröße von 0,2 bis 0,4 mm. Sind 3 Silberschichten im oberen Silberschichtteil vorhanden, so sind mit Bezug auf Gewichtsanteil am Gesamtkatalysator (Korngröße der Teilchen) bevorzugt: obere Silberschicht 4 bis 59 (0,2 bis 0,4 mm); mittlere Silberschicht 4 bis 59 (0,4 bis 0,6 mm); untere Silberschicht 5 bis 60 (0,6 bis 0,75 mm) Gewichtsprozent. Entsprechend sind bei dem mittleren Silberschichtteil mit Bezug auf Gewichtsanteil (Korngröße der Teilchen) bevorzugt:

a) 2 Silberschichten: obere Silberschicht 1 bis 29 (0,75 bis 0,9 mm) Gew.%;
untere Silberschicht 1 bis 29 (0,9 bis 1 mm) Gew.%.

b) 3 Silberschichten: obere Silberschicht 0,7 bis 28,7 (0,75 bis 0,8 mm) Gew.%;
mittlere Silberschicht 0,7 bis 28,7 (0,8 bis 0,9 mm) Gew.%;
untere Silberschicht 0,6 bis 28,6 (0,9 bis 1 mm) Gew.%.

Bei dem unteren Silberschichtteil sind bevorzugt:

c) 2 Silberschichten: obere Silberschicht 15 bis 70 (1 bis 1,75 mm) Gew.%;
untere Silberschicht 15 bis 70 (1,75 bis 2,5 mm) Gew.%.

d) 3 Silberschichten: obere Silberschicht 10 bis 65 (1 bis 1,5 mm) Gew.%;
mittlere Silberschicht 10 bis 65 (1,5 bis 2 mm) Gew.%;
untere Silberschicht 10 bis 65 (2 bis 2,5 mm) Gew.%.

Ein besonders vorteilhafter Katalysator hat die folgenden Zusammensetzungen:

Schicht 1:6,5—61,5 Gew.% des Katalysators mit Teilchen der Krongröße 0,2—0,4 mm (oberste)

Schicht 2: 6,5—61,5 Gew.% des Katalysators mit Teilchen der Korngröße 0,4—0,75 mm

Schicht 3: 1—29 Gew.% des Katalysators mit Teilchen der Korngröße 0,75—0,9 mm

Schicht 4: 1—29 Gew.% des Katalysators mit Teilchen der Korngröße 0,9—1 mm

Schicht 5: 15—70 Gew.% des Katalysators mit Teilchen der Korngröße 1—1,75 mm

Schicht 6: 15—70 Gew.% des Katalysators mit Teilchen der Korngröße 1,75—2,5 mm. (unterste)

Zweckmäßig belastet man den Katalysator mit 0,2 bis 2,5 t, insbensondere 0,6 bis 2 t dampfförmigem Ausgangsstoff II je $m^2$ Katalysatorbettquerschnitt und Stunde. Zur großtechnischen Ausführung verwendet man bevorzugt Katalysatorbettdurchmesser von mindestens 0,05, zweckmäßig 0,1 bis 3 Metern. Verweilzeiten der Umsetzung von 0,001 bis 1, bevorzugt im Falle der Herstellung von Methoxyacetaldehyd im Falle der Ausführungsform a) oder b) 0,01 bis 0,6, im Falle der Herstellung von Methoxyaceton 0,01 bis 0,5 Minuten sind vorteilhaft. Die Verweilzeit wird auf die Reaktionszone ohne Katalysatorfüllung bezogen und so berechnet. Als Berechnungsgrundlage kann z.B. der Reaktionsraum eines leeren Reaktorrohres dienen.

Die Umsetzung wird zweckmäßig bei einer Temperatur von 450 bis 700°C, vorzugsweise bei einer Temperatur von 475 bis 650°C, insbesondere 500 bis 625°C, drucklos oder unter Druck, diskontinuierlich oder vorzugsweise kontinuierlich durchgeführt. Man kann in Abwesenheit zusätzlicher

Lösungsmittel umsetzen; zweckmäßig verwendet man Wasser, vorteilhaft in einer Menge von 5 bis 40, insbesondere von 10 bis 20 Gewichtsprozent, bezogen auf Ausgangsstoff II. Ebenfalls können Nebenstoffe aus der Herstellung der Ausgangsstoffe II noch mit diesen gemischt sein, z.B. in einer Menge bis zu 10 Gewichtsprozent, bezogen auf Ausgansstoff II.

Die Oxidation kann wie folgt durchgeführt werden: In ein Verdampfungsaggregat, z.B. einen Fallstromverdampfer, werden einzeln oder im Gemisch Ausgangstoff II und gegebenenfalls Wasser eingegeben und verdampft, zweckmäßig bei 70 bis 180°C. Anschließend wird das Gasgemisch aus dampfförmigem Ausgangsstoff II, Luft, gegebenenfalls Inertgas und Wasserdampf in vorgenannten Mengen bei der Reaktionstemperatur durch den Katalysator geleitet. Das Verfahren wird im allgemeinen bei Drücken zwischen 0,5 und 3 bar, vorzugsweise zwischen 0,8 und 1,8 bar, kontinuierlich durchgeführt. Zweckmäßig wird vor Beginn des Verfahrens der Silberkatalysator auf einen Temperatur von 250 bis 500,. vorzugsweise von 380 bis 450°C erhitzt. Der Beginn der exothermen Umsetzung wird zweckmäßig festgestellt, indem man Luft dem ausgangsgemisch zugibt und die Temperaturveränderung im Katalysator prüft. Setzt die Reaktion ein, beobachtet man sofort einen Anstieg der Temperatur, anderfalls wird die Temperatur durch die Zuführung der kalten Luft sinken. Die Temperatur wird zweckmäßig im Katalysator durch Thermoelemente gemessen. Ab Beginn der Reaktion leitet man im allgemeinen die Luft kontinuierlich dem dampfförmigen Ausgangsgemisch zu, gegebenenfalls unter Einleiten durch den Sumpf des Verdampfungsaggregates. Es ist vorteilhaft, die die Katalysatorzone verlassenden Reaktionsgase innerhalb kurzer Zeit abzukühlen, z.B. auf Temperaturen von 20 bis 160°C. Die Hauptteil des Endstoffes 1 wird so kondensiert. Das abgekühlte Gasgemisch wird dann zweckmäßig einem Absorptionsturm zugeführt, in welchem der Endstoff I mit einem geeigneten Lösungsmittel wie Dimethylformamid, Dimethylsulfoxid, Aceton, Methanol oder Wasser sowie deren Gemische und/oder in vorgelegtem Kondensat früherer Umsetzungen, vorteilhaft im Gegenstrom, aus dem Gasgemisch gewaschen wird. Aus Kondensat und den Absorbaten wird der Endstoff I dann in üblicher Weise, z.B. durch Destillation, isoliert.

Die nach dem Verfahren der Erfindung herstellbaren veretherten Hydroxyalkdehyde und Hydroxyketone sind wertvolle Ausgangsstoffe für die Herstellung von Farbstoffen, Schädlingsbekämpfungsmitteln, Kunststoffen, Riechstoffen. Bezüglich der Verwendung wird auf die vorgenannten Veröffentlichungen verwiesen.

Die in den folgenden Beispielen angegebenen Teile sind Gewichsteile.

Beispiel 1

*(Zeichnung)*

Man verwendet eine Anlage, bestehend aus einem Verdampfer (1), einem senkrechten Rohrreaktor (2), einem nachgeschalteten Kühler (3) und einer Absorptionsanlage (4). Der Verdampfer ist durch die Leitung (5) mit dem Rohrreaktor verbunden. Die Leitung ist bis zum Reaktor hin beheizbar. Die Katalysatorschicht liegt unterhalb des Reaktorkopfes, weiter unten folgt die Kühlzone (3), die durch die Leitung (7) mit der Absorptionsanlage (4) verbunden ist. Das in der Kühlzone (3) anfallende Kondensat wird über Leitung (6) zur Leitung (11) geführt. Die Absorptionsanlage (4) bilden vier in Kaskade angeordnete Absorptionskolonnen mit Kühlung. Die vier Kolonnen sind mit 15 mm Pallringen aus Keramik gefüllt. Über Leitung (8) entweicht das Abgas.

In den Reaktor (2) wird ein Katalysator (1,63 Teile) aus Silberkristallen (Ag), Kupferkristallen (Cu) sowie Katalysatorteilchen (94,1 Gewichtsprozent Cu, 4,9 Gewichtsprozent Sn, 1 Gewichtsprozent P = Cu/Sn/P) folgender Zusammensetzung eingetragen:

| | Teilchenart | Anteil am Katalysator Gewichtsprozent | Korngröße mm |
|---|---|---|---|
| Schicht 1 (oben) | Cu /Sn /P | 2,7 | 0,2 bis 0,75 |
| Schicht 2 | Cu | 18.8 | 0,2 bis 0,75 |
| Schicht 3 | Ag | 13,2 | 0,2 bis 0,75 |
| Schicht 4 | Ag | 14,7 | 0,75 bis 1,0 |
| Schicht 5 (unten) | Ag | 50,6 | 1,0 bis 2,5 |

Der Durchmesser der Katalysatorschicht beträgt 0,15 Meter, die Gesamtschichtdicke des Katalysators vor Beginn der Reaktion 22 Millimeter.

Der Katalysator wird durch Durchleiten von heißem Stickstoff auf eine Temperatur von 380°C

erhitzt. In dem Verdampfer werden über Leitung (10) 15 Teile Athylenglykolmonomethyläther eingegeben und auf 80°C erwärmt. Nun werden über Leitung (9) in den Verdampfer 0,95 Teile Luft pro Stunde eingeleitet und der Verdampferinhalt weiter bis auf 110°C erwärmt. Danach wird die Luftmenge auf 1,4 Teile pro Stunde gesteigert, wobei die Temperatur des Katalysators zu steigen beginnt. Mit Beginn der Reaktion, erkenntlich durch die steigende Katalysatortemperatur, werden innerhalb von 3 Stunden die Stickstoffzufuhr eingestellt, die Luftmenge auf stündlich 14,6 Teile Luft, und gleichzeitig über Leitung (10), Verdampfer (1) und Leitung (5) die durch den Katalysator geleitete Menge an Äthylenglykolmonomethyläther auf 14,1 Teile pro Stunde (entsprechend einer Katalysatorbelastung von 0,8 Tonnen Äthylenglykolmonomethyläther pro Stunde und Quadratmeter Katalysatorbettquerschnitt) gebracht und 9,3 Teile Wasser pro Stunde zugegeben. Bei einem Druck vor dem Katalysator von 1,04 bar stellt sich eine Katalysatortemperatur von 575°C ein. Das Reaktionsgemisch wird anschließend in der Kühlzone des Reaktors (3) auf 94°C abgekühlt. Die Absorption (4) selbst erfolgt in 4 Stufen im Gegenstrom in Form einer Gaswäsche. Als Absorptionsflüssigkeit werden über Leitung (12) 11,5 Teile Wasser pro Stunde mit einer Temperatur von 25°C in die Kaskade (4) eingeleitet und verwendet. Innerhalb von 720 Stunden werden 10 152 Teile Äthylenglykolmonomethyläther über den Katalysator geführt. Man erhält über Leitung (11) insgesamt 2 274 Teile unumgesetzten Äthylenglykolmonomethyläther und 5 860 Teile Methoxyacetaldehyd. Der Umsatz beträgt 77,6 Prozent, die Ausbeute an Methoxyacetaldehyd 76,4% der Theorie, bezogen auf umgesetzten Äthylenglykolmonomethyläther.

Beispiel 2

Analog Beispiel 1 wird ein Gemisch aus 56,3 Teilen 1,2-Propylenglykolmonomethyläther und 14,1 Teilen Wasser mit 58,5 Teilen Luft innerhalb von 5 Stunden bei 600°C und 1,09 bar über einen Katalysator (0,79 Teile) folgender Zusammensetzung geführt:

| | Teilchenart | Anteil am Katalysator (Gew. %) | Korngröße mm |
|---|---|---|---|
| Schicht 1 (oberste) | Cu | 2,5 | 0,2 bis 0,75 |
| Schicht 2 | Ag | 12 | 0,2 bis 0,4 |
| Schicht 3 | Ag | 12,5 | 0,4 bis 0,75 |
| Schicht 4 | Ag | 6 | 0,75 bis 0,9 |
| Schicht 5 | Ag | 6 | 0,9 bis 1,0 |
| Schicht 6 | Ag | 30 | 1 bis 1,75 |
| Schicht 7 (unterste) | Ag | 31 | 1,75 bis 2,5 |

Man erhält 0,34 Teile unumgesetzten Propylenglykolmonomethyläther und 38,25 Teile Methoxyaceton. Der Umsatz beträgt 99,4 Prozent, die Ausbeute an Methoxyaceton 69,9% der Theorie, bezogen auf umgesetzten Propylenglykolmonomethyläther.

Beispiel 3

Man verwendet eine Anlage, bestehend aus einem Verdampfer, einem mit ihm vorbundenen, senkrechten Rohrreaktor, einem nachgeschalteten Kühler und einer Absorptionsanlage. Die Katalysatorschicht liegt unterhalbt des Reaktorkopfes, weiter unten folgt die Kühlzone, die durch eine Leitung mit der Absorptionsanlage verbunden ist.

In den Reaktor wird ein Katalysator (0,79 Teile) aus Silberkristallen folgender Zusammensetzung eingetragen:

| | Anteil am Katalysator Gew. % | Korngröße mm |
|---|---|---|
| Schicht 1 (oben) | 14,5 | 0,2 bis 0,4 |
| Schicht 2 | 12,5 | 0,4 bis 0,75 |
| Schicht 3 | 6 | 0,75 bis 0,9 |
| Schicht 4 | 6 | 0,9 bis 1,0 |
| Schicht 5 | 30 | 1 bis 1,75 |
| Schicht 6 (unten) | 31 | 1,75 bis 2,5 |

Der Durchmesser der Katalysatorschicht beträgt 0,15 Meter, die Gesamtschichtdicke des Katalysators vor Beginn der Reaktion 22 Millimeter.

In dem Verdampfer werden stündlich 11,26 Teile 1,2-Propylenglykolmonomethyläther und 2,82 Teile Wasser bei 110°C verdampft. Durch den Verdampfer werden 11,7 Teile Luft pro Stunde geleitet. Das Dampf/Luft-Gemisch wird über den katalysator geleitet. Die Katalysatorbelastung beträgt 0,64 Tonnen 1,2-Propylenglykolmonomethyläther pro Stunde und Quadratmeter Katalysatorbettquerschnitt. Bei einem Druck vor dem Katalysator von 1,03 bar und einer Katalysatortemperatur von 600°C wird die Umsetzung durchgeführt. Das Reaktionsgemisch wird anschließend in der Kühlzone des Reaktors auf 40°C abgekühlt. Die Absorption selbst erfolgt in 4 Stufen im Gegenstrom in Form einer Gaswäsche. Als Absorptionsflüssigkeit werden 11,5 Teile Wasser pro Stunde mit einer Temperatur von 25°C in die Absorptionsanlage eingeleitet und verwendet. Man erhält stündlich insgesamt 0,068 Teile unumgesetzten 1,2-Propylenglykolmonomethyläther und 9,84 Teile Methoxyaceton. Der Umsatz beträgt 99,4 Prozent, die Ausbeute an Methoxyaceton 89,4% der Theorie, bezogen auf eingesetzten 1,2-Propylenglykolmonomethyläther.

## Patentansprüche

Verfahren zur Herstellung von aliphatischen, mit aliphatischen Gruppen verätherten Hydroxycarbonylverbindungen durch katalytische Oxidation von Hydroxyalkoholen in Gegenwart von Metallkatalysatoren, dadurch gekennzeichnet, daß man aliphatische, mit aliphatischen Gruppen verätherte Hydroxyalkohole in Gegenwart eines Katalysators mit der Gesamtschichtdicke von 5 bis 100 Millimeter und

a) 3 oder mehr Schichten Silberkristallen, wobei ein Teil der Schichten Silberkristalle 30 bis 85 Gewichtsprozent des Katalysators mit Teilchen der Korngröße 1 bis 2,5 mm, ein Teil der Schichten Silberkristalle 2 bis 30 Gewichtsprozent des Katalysators mit Teilchen der Korngröße 0,75 bis 1 mm und der restliche Teile der Schichten Silberkristalle 8 bis 50 Gewichtsprozent des Katalysators mit Teilchen der Korngröße 0,2 bis 0,75 mm enthalten, und einer oder mehr Schichten Kupferkristallen, die 1 bis 40 Gewichtsprozent des Katalysators mit Teilchen der Korngröße 0,2 bis 0,75 mm enthalten, oder

b) 3 oder mehr Schichten Silberkristallen, wobei ein Teil der Schichten Silberkristalle 30 bis 85 Gewichtsprozent des Katalysators mit Teilchen der Korngröße 1 bis 2,5 mm, ein Teil der Schichten Silberkristalle 2 bis 30 Gewichtsprozent des Katalysators mit Teilchen der Korngröße 0,75 bis 1 mm und der restliche Teil der Schichten Silberkristalle 8 bis 50 Gewichtsprozent des Katalysators mit Teilchen der Korngröße 0,2 bis 0,75 mm enthalten, und einer oder mehr Schichten Kupferkristallen, die 0,5 bis 40 Gewichtsprozent des Katalysators mit Teilchen der Korngröße 0,2 bis 0,75 mm enthalten, und einer oder mehr Schichten von Katalysatorteilchen mit 70 bis 99 Gewichtsprozent Kupfer, 0,9 bis 20 Gewichtsprozent Zinn und 0,1 bis 10 Gewichtsprozent Phosphor, bezogen auf das Gewicht dieser Teilchen an diesen Elementen, wobei diese Schichten Katalysatorteilchen 0,5 bis 10 Gewichtsprozent des Katalysators mit Teilchen der Korngröße 0,2 bis 0,75 mm enthalten, oder

c) 3 oder mehr Schichten Silberkristallen, wobei ein Teil der Schichten Silberkristalle 30 bis 85 Gewichtsprozent des Katalysators mit Teilchen der Korngröße 1 bis 2,5 mm, ein Teil der Schichten Silberkristalle 2 bis 30 Gewichtsprozent des Katalysators mit Teilchen der Korngröße 0,75 bis 1 mm und der restliche Teil der Schichten Silberkristalle 13 bis 68 Gewichtsprozent des Katalysators mit Teilchen der Korngröße 0,2 bis 0,75 mm enthalten,

bei einer Temperatur von 450 bis 700°C oxidiert.

## Claim

A process for the preparation of an aliphatic hydroxycarbonyl compound etherified with an aliphatic group, by catalytic oxidation of a hydroxyalcohol in the presence of a metal catalyst, characterised in that an aliphatic hydroxyalcohol, etherified with an aliphatic group, is oxidized, at from 450 to 700°C, in the presence of a catalyst which has a total thickness of from 5 to 100 millimeters and comprises

a) 3 or more layers of silver crystals, with some of the layers of silver crystals containing particles of size from 1 to 2.5 mm and constituting from 30 to 85 percent by weight of the catalyst, some of the layers of silver crystals containing particles of size from 0.75 to 1 mm and constituting from 2 to 30 percent by weight of the catalyst and the remainder of the layers of silver crystals containing particles of size from 0.2 to 0.75 mm and constituting from 8 to 50 percent by weight of the catalyst, and one or more layers of copper crystals containing particles of size from 0.2 to 0.75 mm and constituting from 1 to 40 percent by weight of the catalyst, or

b) 3 or more layers of silver crystals, with some of the layers of silver crystals containing particles of size from 1 to 2.5 mm and constituting from 30 to 85 percent by weight of the catalyst, some of the layers of silver crystals containing particles of size from 0.75 to 1 mm and constituting from 2 to 30 percent by weight of the catalyst and the remainder of the layers of silver crystals containing particles of size from 0.2 to 0.75 mm and constituting from 8 to 50 percent by weight of the catalyst, and one or more layers of copper crystals containing particles of size from 0.2 to 0.75 mm and constituting from 0.5 to 40 percent by weight of the catalyst, and one or more layers of catalyst particles comprising from 70 to 99 percent by weight of copper, from 0.9 to 20 percent by weight of tin and from 0.1 to 10 percent by weight of phosphorus, based on the total weight of these elements in these particles, with these layers of catalyst particles containing particles of size from 0.2 to 0.75 mm and constituting from 0.5 to 10 percent by weight of the catalyst, or

c) 3 or more layers of silver crystals, with some of the layers of silver crystals containing particles of size from 1 to 2.5 mm and constituting from 30 to 85 percent by weight of the catalyst, some of the layers of silver crystals containing particles of size from 0.75 to 1 mm and constituting from 2 to 30 percent by weight of the catalyst and the remainder of the layers of silver crystals containing particles of size from 0.2 to 0.75 mm and constituting from 13 to 68 percent by weight of the catalyst.

## Revendication

Procédé pour la préparation de composés hydroxycarbonylés aliphatiques, étherifiés avec des groupes aliphatiques, par oxydation catalytique d'hydroxyalcools en présence de catalyseurs métalliques, caractérisé en ce qu'on oxyde à une température de 450 à 700°C des hydroxyalcools aliphatiques, éthérifiés avec des groups aliphatiques, en présence d'un catalyseur ayant une épaisseur de couche totale de 5 à 100 mm et comprenant:

a) 3 couches ou plus de cristaux d'argent, une partie des couches de cristaux d'argent contenant 30 à 85% en poids du catalyseur, avec des particules d'une grosseur de grains de 1 à 2,5 mm, une partie des couches de cristaux d'argent contenant 2 à 30% en poids du catalyseur, avec des particules d'une grosseur de grains de 0,75 à 1 mm et le reste des couches de cristaux d'argent contenant 8 à 50% en poids du catalyseur, avec des particules d'une grosseur de grains de 0,2 à 0,75 mm, ainsi qu'une ou plusieurs couches de cristaux de cuivre qui contiennent 1 à 40% en poids du catalyseur, avec des particules d'une grosseur de grains de 0,2 à 0,75 mm, ou

b) 3 couches ou plus de cristaux d'argent, une partie des couches de cristaux d'argent contenant 30 à 85% en poids du catalyseur, avec des particules d'une grosseur de grains de 1 à 2,5 mm, une partie des couches de cristaux d'argent contenant 2 à 30% en poids du catalyseur, avec des particules d'une grosseur de grains de 0,75 à 1 mm et le reste des couches de cristaux d'argent contenant 8 à 50% en poids du catalyseur, avec des particules d'une grosseur de grains de 0,2 à 0,75 mm, et une ou plusieurs couches de cristaux de cuivre qui contiennent 0,5 à 40% en poids du catalyseur, avec des particules d'une grosseur de grains de 0,2 à 0,75 mm, ainsi qu'une ou plusieurs couches de particules de catalyseur formées de 70 à 99% en poids de cuivre, 0,9 à 20% en poids d'étain et 0,1 à 10% en poids de phosphore, sur la base du poids de ces particules en ces éléments, ces couches de particules de catalyseur contenant 0,5 à 10% en poids du catalyseur, avec des particules d'une grosseur de grains de 0,2 à 0,75 mm, ou

c) 3 couches ou plus de cristaux d'argent, une partie des couches de cristaux d'argent contenant 30 à 85% en poids du catalyseur, avec des particules d'une grosseur de grains de 1 à 2,5 mm, une partie des couches de cristaux d'argent contenant 2 à 30% en poids du catalyseur, avec des particules d'une grosseur de grains de 0,75 à 1 mm et le reste des couches de cristaux d'argent contenant 13 à 68% en poids du catalyseur, avec des particules d'une grosseur de grains de 0,2 à 0,75 mm.

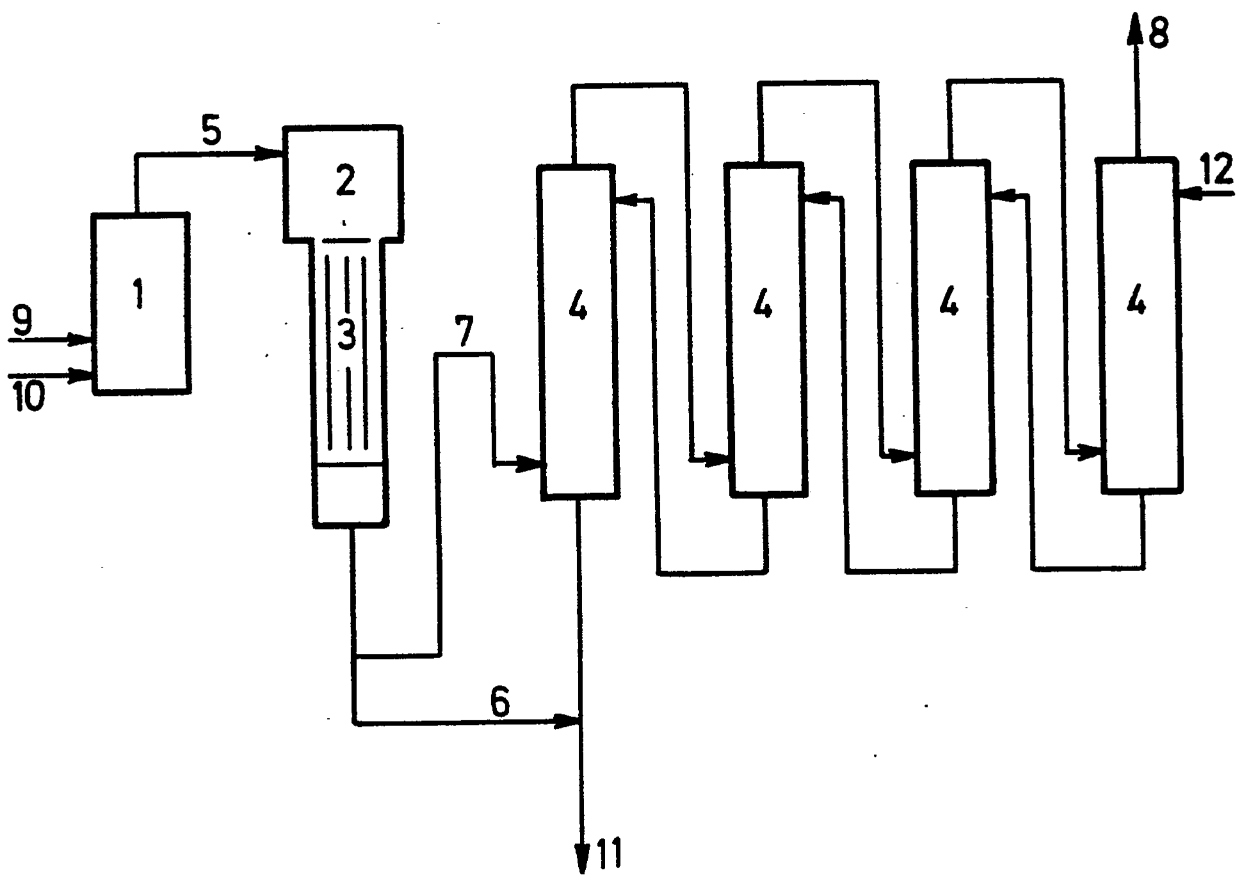
1
2
3
4
4
4
4
5
6
7
8
9
10
11
12